# EUROPÄISCHE PATENTSCHRIFT

(11) **EP 1 865 921 B1**
(45) Veröffentlichungstag und Bekanntmachung des Hinweises auf die Patenterteilung: **04.12.2013**
(21) Anmeldenummer: 06723580.4
(22) Anmeldetag: 21.03.2006
(51) Int. Cl.: A61K 9/20, A61K 9/28, A61K 31/426, C07D 417/12

(54) **PHARMAZEUTISCHE ZUBEREITUNG VON N-[5-(AMINOSULFONYL)-4-METHYL-1,3-THIAZOL-2-YL]-N-METHYL-2-[4-(2-PYRIDINYL)PHENYL]ACETAMID**
PHARMACEUTICAL PREPARATION OF N-[5-(AMINOSULFONYL)-4-METHYL-1,3-THIAZOL-2-YL]-N-METHYL-2-[4-(2-PYRIDINYL)PHENYL]ACETAMIDE
PRÉPARATION PHARMACEUTIQUE DE N-[5-(AMINOSULFONYL)-4-MÉTHYL-1,3-THIAZOL-2-YL]-N-MÉTHYL-2-[4-(2-PYRIDINYL)PHÉNYL]ACÉTAMIDE

(30) Priorität: 30.03.2005 DE 102005014248
(43) Veröffentlichungstag der Anmeldung: 19.12.2007
(73) Patentinhaber: AiCuris GmbH & Co. KG, 42117 Wuppertal (DE)
(72) Erfinder: LAICH, Tobias, 51069 Köln (DE); LIEBELT, Katrin, 79106 Freiburg (DE)
(74) Vertreter: Kilger, Ute
(86) Internationale Anmeldenummer: PCT/EP2006/002566
(87) Internationale Veröffentlichungsnummer: WO 2006/103011

(56) Entgegenhaltungen:
- WO-A-2004/078163
- WO-A1-01/47904
- DE-A1- 10 129 716
- DE-A1- 10 131 128
- AULTON M E (ED): 'Pharmaceutics: The science of dosage form design', Bd. 2, 2002, CHURCHILL LIVINGSTONE, EDINBURGH, ISBN 9780443055171 vol. 'Solubility - Salts', Seiten 115 - 118

## Beschreibung

Die vorliegende Erfindung betrifft eine pharmazeutische Zubereitung zur oralen Anwendung, die N-[5-(Aminosulfonyl)-4-methyl-1,3-thiazol-2-yl]-N-methyl-2-[4-(2-pyridinyl)phenyl]acetamid oder dessen Hydrate und/oder Solvate sowie eine Säure enthält, ein Verfahren zu ihrer Herstellung, sowie die Verwendung dieser Zubereitung zur Behandlung und/oder Prophylaxe von Erkrankungen, die durch Herpes-Viren, insbesondere Erkrankungen, die durch Herpes Simplex Viren hervorgerufen werden.

N-[5-(Aminosulfonyl)-4-methyl-1,3-thiazol-2-yl]-N-methyl-2-[4-(2-pyridinyl)phenyl]acetamid ist eine gegen Erkrankungen, die durch Herpes Simplex Viren hervorgerufen werden, hochwirksame Verbindung der folgenden Formel: 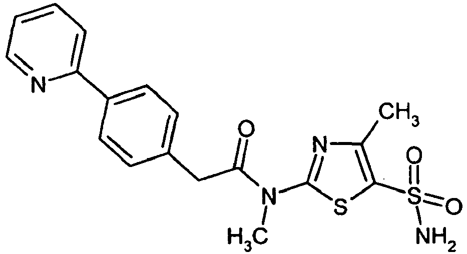 N-[5-(Aminosulfonyl)-4-methyl-1,3-thiazol-2-yl]-N-methyl-2-[4-(2-pyridinyl)phenyl]acetamid

Sie ist erstmals beschrieben in der WO 01/47904. Wenn im folgenden vom Wirkstoff (I) die Rede ist, so sind dabei alle Modifikationen von N-[5-(Aminosulfonyl)-4-methyl-1,3-thiazol-2-yl]-N-methyl-2-[4-(2-pyridinyl)-phenyl]acetamid sowie die Hydrate und/oder Solvate mit umfasst.

Aus der DE 101 31 128 A1 sind weitere Sulfonamide mit antiviraler Wirkung bekannt.

Aus der DE 101 29 716 A1 sind Kombinationspräparate zur Herpesbehandlung bekannt, die u.a. Thiazolylsulfonamide in Kombination mit Acetylsalicylsäure aufweisen.

Aus der WO 2004/078163 A sind pharmazeutische Zubereitungen bekannt, die Co-Kristalle aus einem Wirkstoff und einem Co-Kristallbildner aufweisen, wobei der Wirkstoff zumindest eine gegebene funktionelle Gruppe aufweist, so dass der Wirkstoff und der Co-Kristallbildner in der Lage sind, unter Kristallisationsbedingungen aus einer Lösung zu co-kristallisieren.

Für den Wirkstoff (I) werden für die Therapie von Herpes Erkrankungen beim Menschen schnellfreisetzende und lagerstabile Tabletten benötigt. Schnellfreisetzend bedeutet hier, dass mindestens 80 % der Wirkstoffmenge innerhalb von 30 Minuten in zum Beispiel 0.1N Salzsäure bei 37°C freigesetzt werden. Lagerstabilität ist gekennzeichnet durch unveränderte Freisetzungseigenschaften des Wirkstoffs (I) nach einer Lagerung der Tablette über mehrere Wochen bzw. Monate unter definierten Klimabedingungen (Temperatur, Luftfeuchte) und durch die chemische Stabilität des Wirkstoffs.

Dazu wird üblicherweise der Wirkstoff zusammen mit gängigen pharmazeutischen Hilfsstoffen gemischt, eventuell granuliert und anschließend zu Tabletten verpresst. Typische Hilfsstoffklassen sind Sprengmittel (Zerfallshilfsmittel), Füllstoffe, Bindemittel, Schmiermittel und Tenside. Diese können einzeln oder in beliebiger Kombination eingesetzt werden, um die gewünschte rasche Wirkstofffreisetzung und Lagerstabilität aus der Zubereitung (Tablette) zu erhalten.

Wendet man diese im Stand der Technik bekannten Zusammensetzungen jedoch auf den hier beschriebenen Wirkstoff (I) an, zeigt sich unerwartet, dass es nicht zu einer sofortigen Wirkstofffreisetzung kommt, sondern zu einer deutlich verzögerten Freisetzung, die therapeutisch nicht erwünscht ist.

Ursache für dieses Freisetzungsverhalten ist eine "Verklumpung" bzw. Gelbildung des Wirkstoffs (I), die sich in extremer Weise bei Freisetzungsversuchen mit reinem Wirkstoff (I) zeigt, aber auch als Ursache bei der Freisetzung von Tabletten vermutet werden kann.

Der Zusatz von sauren Hilfsstoffen (Säurekomponenten) zu pharmazeutischen Zusammensetzungen, insbesondere Tabletten, ist prinzipiell bekannt. So wird beispielsweise Zitronensäure häufig als Stabilisierungsmittel (Komplexbildung, Veränderung des pH-Wertes) oder als Zerfallhilfsmittel in Brausetabletten eingesetzt. Andere Säuren wie Sorbinsäure, Propionsäure oder Benzoesäure sind als Konservierungsmittel gebräuchlich, finden sich aber üblicherweise nicht in Tabletten. Manche Säuren wie beispielsweise Stearinsäure eigenen sich als Schmiermittel oder Emulgatoren. Daneben gibt es polymere Säuren, wie Polyacrylsäure oder Polylactide, die ebenfalls als pharmazeutische Hilfsstoffe eingesetzt werden.

Bislang nicht beschrieben ist der Einsatz von Säuren ohne Zusatz von gasentwickelnden Substanzen, wie Natriumcarbonat, zur Beschleunigung der Wirkstofffreisetzung in Kombination mit einer Verbesserung der Stabilität.

Der Erfindung lag die Aufgabe zugrunde, eine pharmazeutische Formulierung bereitzustellen, aus denen Tabletten mit ausreichender Freisetzungsgeschwindigkeit des Wirkstoffs (I) hergestellt werden können, und die zur selben Zeit über ausgezeichnete Lagerstabilität verfügen.

Es wurde überraschend gefunden, dass die zuvor beschriebene Aufgabenstellung gelöst werden kann durch eine pharmazeutische Formulierung, die neben gängigen pharmazeutischen Hilfsstoffen eine Säure in einem bestimmten Mengenanteil enthält. Der Zusatz von gasentwickelnden Substanzen ist nicht erforderlich.

Der Zusatz der Säure erfolgt während der Herstellung der pharmazeutischen Zubereitung. Gute Freisetzungsgeschwindigkeiten des Wirkstoffs (I) aus der Tablette kommen besonders gut bei gemeinsamer Feuchtgranulation des Wirkstoffs (I) mit der Säure zustande.

Weiterhin wurde überraschend gefunden, dass die Freisetzungsgeschwindigkeit des Wirkstoffs (I) und die Lagerstabilität von der verwendeten Säure und von dem Mengenanteil der Säure abhängig sind.

Gegenstand der vorliegenden Erfindung sind somit pharmazeutische Zubereitungen zur oralen Anwendung, die N-[5-(Aminosulfonyl)-4-methyl-1,3-thiazol-2-yl]-N-methyl-2-[4-(2-pyridinyl)-phenyl]acetamid oder dessen Hydrate und/oder Solvate, einen oder mehrere gängige pharmazeutische Hilfsstoffe und Methansulfonsäure enthalten, wobei das Acetamid und die Säure ein Salz bilden.

Bei der erfindungsgemäßen pharmazeutischen Zubereitung handelt es sich um eine Zubereitung zur oralen Anwendung.

Gegenstand dieser Erfindung ist weiterhin ein Verfahren zur Herstellung der pharmazeutischen Zubereitung, dadurch gekennzeichnet, dass die Säure während der Herstellung der pharmazeutischen Zubereitung zugesetzt wird, bevorzugt wird der Wirkstoff (I) mit der Säure in einer gemeinsamen Feuchtgranulation gemischt.

Des Weiteren ist ein Verfahren zur Herstellung von Tabletten, die solche Zubereitungen enthalten, Gegenstand dieser Erfindung.

Des Weiteren sind Tabletten, die eine Lagerstabilität von ein bis sechs Jahren, bevorzugt von zwei bis fünf Jahren aufweisen, beschrieben.

Die erfindungsgemäße Formulierung umfasst bevorzugt von 30 bis 90%, besonders bevorzugt 50 bis 70% N-[5-(Aminosulfonyl)-4-methyl-1,3-thiazol-2-yl]-N-methyl-2-[4-(2-pyridinyl)phenyl]-acetamid oder Hydrate und/oder Solvate davon (Alle %-Angaben sind Gewichtsprozente bezogen auf das Gewicht der pharmazeutischen Zubereitungen).

Die pharmazeutische Zubereitung enthält bezogen auf die einzelne Tablette im allgemeinen von 30 bis 1200 mg Wirkstoff (I), bevorzugt von 50 bis 500 mg, besonders bevorzugt von 50 bis 250 mg.

Als zur Erreichung der erfindungsgemäßen Aufgabenstellung wesentlichen Bestandteil enthält die pharmazeutische Zubereitung ein stöchiometrisches Verhältnis von Wirkstoff (I) zu Säure von 1 zu 0.10 bis 1.10, bevorzugt von 1 zu 0.50 bis 1.00 und besonders bevorzugt von 1 zu 0.90 bis 0.98.

Die erfindungsgemäße pharmazeutische Zubereitung enthält gegebenenfalls ein oder mehrere Trockenbindemittel, das/die beispielsweise ausgewählt wird/werden aus der Gruppe die besteht aus: mikrokristalliner Cellulose, Fasercellulose, Calciumphosphaten und Mannit. Bevorzugt wird erfindungsgemäß mikrokristalline Cellulose verwendet. Diese ist im Handel beispielsweise unter der Bezeichnung Avicel^{®} erhältlich. Die pharmazeutische Zubereitung enthält zweckmäßig 1 bis 20%, bevorzugt 1 bis 10%, besonders bevorzugt 1 bis 5% des oder der Trockenbindemittel.

Die erfindungsgemäße pharmazeutische Zubereitung enthält mindestens ein Zerfallhilfsmittel, das beispielsweise ausgewählt wird aus der Gruppe, die besteht aus Stärke, vorverkleisterte Stärke, Stärkeglykolate, quervernetztes Polyvinylpyrrolidon, Natriumcarboxymethylcellulose (= Cros-carmellose-Natrium) und weitere Salze von Carboxymethylcellulose. Es kann auch eine Mischung aus zwei Zerfällsmitteln Verwendung finden. Erfindungsgemäß ist besonders die Verwendung von Croscarmellose-Natrium und quervernetztes Polyvinylpyrrolidon oder eine Mischung aus beiden bevorzugt. Die pharmazeutische Zubereitung enthält zweckmäßig 3 bis 35%, bevorzugt 10 bis 30%, besonders bevorzugt 15 bis 29% des oder der Zerfallhilfsmittel.

Die pharmazeutische Zubereitung der Erfindung enthält mindestens ein Schmiermittel, das ausgewählt wird aus der Gruppe der Fettsäuren und deren Salze. Erfindungsgemäß besonders bevorzugt ist die Verwendung von Magnesiumstearat. Das Schmiermittel wird zweckmäßig in einer Menge von 0.1 bis 2.0%, besonders bevorzugt von 0.2 bis 1.5% und am meisten bevorzugt von 0.5 bis 1.1% eingesetzt.

Die pharmazeutische Zubereitung der Erfindung enthält gegebenenfalls ein Netzmittel, das ausgewählt wird aus der Gruppe der Tenside und deren Salze. Erfindungsgemäß besonders bevorzugt ist die Verwendung von Tween 80. Das Netzmittel wird zweckmäßig in einer Menge von 0.3 bis 2.0%, besonders bevorzugt von 0.4 bis 1.5% und am meisten bevorzugt von 0.4 bis 0.5% eingesetzt.

Eine besonders bevorzugte pharmazeutische Zubereitung der Erfindung enthält:
von 50 bis 70% N-[5-(Aminosulfonyl)-4-methyl-1,3-thiazol-2-yl]-N-methyl-2-[4-(2-pyridinyl)-phenyl]acetamid oder Hydrate und/oder Solvate davon,
von 15 bis 29% Croscarmellose-Natrium und quervernetztes Polyvinylpyrrolidon (4:1),
von 0.5 bis 1.1% Magnesiumstearat,
von 1 bis 5% mikrokristalline Cellulose,
von gegebenenfalls 0.4 bis 0.5% Tween 80 sowie
von Methansulfonsäure mit einem stöchiometrischen Verhältnis von Acetamid zu Säure von 1 zu 0.90 bis 0.98, wobei das Acetamid und die Säure ein Salz bilden.

Die pharmazeutische Zubereitung der Erfindung kann zweckmäßig durch ein Verfahren hergestellt werden, bei dem N-[5-(Aminosulfonyl)-4-methyl-1,3-thiazol-2-yl]-N-methyl-2-[4-(2-pyridinyl)-phenyl]acetamid, dessen Hydrat und/oder Solvat, gegebenenfalls ein oder mehrere Trockenbindemittel und eine Säure wässrig granuliert werden, das Granulat anschließend mit mindestens einem Zerfallhilfsmittel und mindestens einem Schmiermittel und gegebenenfalls einem Netzmittel vermischt und gegebenenfalls tablettiert und lackiert wird.

Zur Granulation-werden Verfahren nach dem Prinzip der Schnellmischergranulation verwendet. Die Granulation kann mit Wasser ohne Zusatz eines Bindemittels erfolgen.

Die pharmazeutische Zubereitung der vorliegenden Erfindung wird besonders bevorzugt in Form einer Tablettenformulierung angewendet, die gegebenenfalls lackiert sein kann (Wie bereits oben erwähnt, beziehen sich die Gewichtsangaben in der vorliegenden Patentanmeldung auf das Gesamtgewicht der pharmazeutischen Zubereitung ohne das Gewicht der wahlweise vorhandenen Lackierung). Zur Lackierung können in der pharmazeutischen Technologie übliche Lackierungsformulierungen angewendet werden, wie z.B. solche auf Basis von Hydroxypropylmethylcellulose (HPMC) und/oder Polyethylenglykol verschiedener Molekulargewichte. Weiterhin kann die Lackierung übliche Pigmente oder Farbstoffe enthalten, wie z. B. Titandioxid oder Eisenoxid.

Die erfindungsgemäße pharmazeutische Zubereitung wird bevorzugt zur Behandlung und/oder Prophylaxe von Erkrankungen, die durch Herpes-Viren, insbesondere Erkrankungen, die durch Herpes Simplex Viren hervorgerufen werden, verwendet.

### Beispiele

Die Herstellung der Tabletten erfolgt jeweils im Labormaßstab unter vergleichbaren Granulationsund Tablettierbedingungen.

Es werden zunächst Granulate bzw. Pulvermischungen hergestellt. Diese werden auf üblichen Tablettenpressen zu Tabletten mit einer sich aus dem Beispiel ergebenden Gesamtmasse mit einem Preßwerkzeug im Format 6 mm, Wölbungsradius 9 mm verpresst. Die Bruchfestigkeit der Tabletten in einem gängigen Testgerät (z.B. Schleuniger 6D) sollte um 60N liegen.

Gegebenenfalls können die Tabletten auch mit einem Lack überzogen werden. Hierzu kann beispielsweise eine wässrige 3.5 %-ige (w/w) Suspension (Feststoffe: 60% Hydroxypropyl-methylcellulose der Viskosität 15cp, 20% Polyethlenglycol 400 und 20% Pigmenten, wie z.B. Eisenoxiden und oder Titandioxid) eingesetzt werden. Die hierzu notwendigen Verfahrensschritte und Apparate sind dem Fachmann hinreichend bekannt. Angaben zu Lackmengen in den Beispielen beziehen sich auf mg Lackfeststoff pro Tablette, ebenso die Mengenangaben in den Beispielrezepturen (mg Feststoff pro Tablette).

Zur Herstellung von 85 g Tabletten nach Beispiel 1 werden die Mengen entsprechend der Ansatzgröße hochgerechnet und die Feststoffe gemischt. Zum Mischen sind beispielsweise 15 min in einem 1 Liter Mischgefäß in einem Turbula Mischer (Fa. Bachofen; Schweiz) ausreichend. Die Mischung wird wie beschrieben zu Tabletten verpresst und die Tabletten können ggf. lackiert werden.

Zur Herstellung von jeweils 50 g Granulat der Beispiele 2 bis 8 werden die Komponenten entsprechend der Ansatzgröße in der angegebenen Zusammensetzung eingewogen. Dabei werden zur Granulation pro Tablette für die Beispiel 2 bis 4 je 42.5 mg Wasser pro Tablette, für die Beispiele 5, 6 und 8 60 mg Wasser pro Tablette und für das Beispiel 7 80 mg Wasser pro Tablette eingesetzt. Das Polyvinylpyrrolidon wird in der Wassermenge gelöst, ebenso wie ggf. die Tenside oder die Säurekomponenten. Alle weiteren Komponenten, mit Ausnahme des Magnesiumstearates, werden mit dieser Lösung granuliert. Dies wird in einer Reibschale geeigneter Größe durchgeführt. Die Zugabe der Granulationslösung erfolgt portionsweise. Im Anschluß wird die feuchte Masse durch ein 2 mm Laborsieb gedrückt und im Trockenschrank bei 60 bis 80°C getrocknet. Das trockene Granulat wird mit der notwendigen Menge Magnesiumstearat gemischt (5 min, 11 Gefäß, Turbula Mischer). Die Mischung wird wie beschrieben zu Tabletten verpresst und die Tabletten können gegebenenfalls lackiert werden.

Unter "mikronisierter Wirkstoff" wird kristalliner Wirkstoff verstanden, der mikronisiert ist. Die Teilchengröße liegt dabei bei 1.04 µm (Lower x 10), 2.97 µm (Middle x 50) und 6.29 µm (Upper x 90).

### Beispiel-1 (Vergleichsbeispiel)

Standard-Tablette enthaltend 50 mg N-[5-(Aminosulfonyl)-4-methyl-1,3-thiazol-2-yl]-N-methyl-2-[4-(2-pyridinyl)phenyl]acetamid als kristalliner Wirkstoff, Wirkstoffgehalt ca. 59% (bezogen auf unlackierte Tablette):

| | |
|---|---|
| N-[5-(Aminosulfonyl)-4-methyl-1,3-thiazol-2-yl]-N-methyl-2-[4-(2-pyridinyl)-phenyl]acetamid, kristallin | 50.00 mg |
| Mikrokristalline Cellulose | 10.15 mg |
| Croscarmellose Natrium | 24.00 mg |
| Magnesiumstearat | 0.85 mg |
| gegebenenfalls HPMC Lack | 2.70 mg |

### Beispiel 2

Tablette enthaltend 50 mg N-[5-(Aminosulfonyl)-4-methyl-1,3-thiazol-2-yl]-N-methyl-2-[4-(2-pyridinyl)-phenyl]acetamid als mikronisierter Wirkstoff, Wirkstoffgehalt ca. 59% (bezogen auf unlackierte Tablette) und Methansulfonsäure in einem molaren Verhältnis von 5%:

| | | |
|---|---|---|
| N-[5-(Aminosulfonyl)-4-methyl-1,3-thiazol-2-yl]- N-methyl-2-[4-(2-pyridinyl)-phenyl]acetamid, mikronisiert | 50.00 mg | (0.124 mmol) |
| Polyvinylpyrrolidon 25 | 3.50 mg | |
| Mikrokristalline Cellulose | 20.05 mg | |
| Methansulfonsäure | 0.60 mg | (0.006 mmol) |
| Croscannellose Natrium | 10.00 mg | |
| Magnesiumstearat | 0.85 mg | |
| gegebenenfalls HPMC Lack | 3.00 mg | |

### Beispiel 3

Tablette enthaltend 50 mg N-[5-(Aminosulfonyl)-4-methyl-1,3-thiazol-2-yl]-N-methyl-2-[4-(2-pyridinyl)-phenyl]acetamid als mikronisierter Wirkstoff, Wirkstoffgehalt ca. 59% (bezogen auf unlackierte Tablette) und Methansulfonsäure in einem molaren Verhältnis von 10%:

| | | |
|---|---|---|
| N-[5-(Aminosulfonyl)-4-methyl-1,3-thiazol-2-yl]- N-methyl-2-[4-(2-pyridinyl)-phenyl]acetamid, mikronisiert | 50.00 mg | (0.124 mmol) |
| Polyvinylpyrrolidon 25 | 3.50 mg | |
| Mikrokristalline Cellulose | 19.46 mg | |
| Methansulfonsäure | 1.19 mg | (0.012 mmol) |
| Croscarmellose Natrium | 10.00 mg | |
| Magnesiumstearat | 0.85 mg | |
| gegebenenfalls HPMC Lack | 3.00 mg | |

### Beispiel 4

Tablette enthaltend 50 mg N-[5-(Aminosulfonyl)-4-methyl-1,3-thiazol-2-yl]-N-methyl-2-[4-(2-pyridinyl)-phenyl]acetamid als mikronisierter Wirkstoff, Wirkstoffgehalt ca. 59% (bezogen auf unlackierte Tablette) und Methansulfonsäure in einem molaren Verhältnis von 50%:

| | | |
|---|---|---|
| N-[5-(Aminosulfonyl)-4-methyl-1,3-thiazol-2-yl]-N-methyl-2-[4-(2-pyridinyl)-phenyl]acetamid, mikronisiert | 50.00 mg | (0.124 mmol) |
| Polyvinylpyrrolidon 25 | 3.50 mg | |
| Mikrokristalline Cellulose | 14.70 mg | |
| Methansulfonsäure | 5.95 mg | (0.062 mmol) |
| Croscarmellose Natrium | 10.00 mg | |
| Magnesiumstearat | 0.85 mg | |
| gegebenenfalls HPMC Lack | 3.00 mg | |

### Beispiel 5

Tablette enthaltend 50 mg N-[5-(Aminosulfonyl)-4-methyl-1,3-thiazol-2-yl]-N-methyl-2-[4-(2-pyridinyl)-phenyl]acetamid als mikronisierter Wirkstoff, Wirkstoffgehalt ca. 56% (bezogen auf unlackierte Tablette) und Methansulfonsäure in einem molaren Verhältnis von 95%:

| | |
|---|---|
| N-[5-(Aminosulfonyl)-4-methyl-1,3-thiazol-2-yl]-N-methyl-2-[4-(2-pyridinyl)-phenyl]acetamid, mikronisiert | 50.00 mg (0.124 mmol) |
| Polyvinylpyrrolidon 25 | 5.00 mg |
| Mikrokristalline Cellulose | 1.41 mg |
| Methansulfonsäure | 11.30 mg (0.118 mmol) |
| Croscärmellose Natrium | 20.00 mg |
| Magnesiumstearat | 0.89 mg |
| Tween 80 | 0.40 mg |
| gegebenenfalls HPMC Lack | 3.00 mg |

### Beispiel 6 (Vergleichsbeispiel)

Tablette enthaltend 50 mg N-[5-(Aminosulfonyl)-4-methyl-1,3-thiazol-2-yl]-N-methyl-2-[4-(2-pyridinyl)-phenyl]acetamid als mikronisierter Wirkstoff, Wirkstoffgehalt ca. 60% (bezogen auf unlackierte Tablette) und Schwefelsäure (95%ig) in einem molaren Verhältnis von 47% (zweiprotonige Säure, d.h. effektiv 94%):

| | |
|---|---|
| N-[5-(Aminosulfonyl)-4-methyl-1,3-thiazol-2-yl]-N-methyl-2-[4-(2-pyridinyl)-phenyl]acetamid, mikronisiert | 50.00 mg (0.124 mmol) |
| Polyvinylpyrrolidon 25 | 5.00 mg |
| Mikrokristalline Cellulose | 1.75 mg |
| Schwefelsäure (95%ig) | 6.00 mg (0.058 mmol) |
| Croscarmellose Natrium | 20.00 mg |
| Magnesiumstearat | 0.85 mg |
| Tween 80 | 0.40 mg |
| gegebenenfalls HPMC Lack | 3.00 mg |

### Beispiel 7 (Vergleichsbeispiel)

Tablette enthaltend 50 mg N-[5-(Aminosulfonyl)-4-methyl-1,3-thiazol-2-yl]-N-methyl-2-[4-(2-pyridinyl)-phenyl]acetamid als mikronisierter Wirkstoff, Wirkstoffgehalt ca. 57% (bezogen auf unlackierte Tablette) und Milchsäure (90%ig) in einem molaren Verhältnis von 94%:

| | |
|---|---|
| N-[5-(Aminosulfonyl)-4-methyl-1,3-thiazol-2-yl]-N-methyl-2-[4-(2-pyridinyl)-phenyl]acetamid, mikronisiert | 50.00 mg (0.124 mmol) |
| Polyvinylpyrrolidon 25 | 5.00 mg |
| Mikrokristalline Cellulose | 0.00 mg |
| Milchsäure (90%ig) | 11.70 mg (0.117 mmol) |
| Croscarmellose Natrium | 20.00 mg |
| Magnesiumstearat | 0.90 mg |
| Tween 80 | 0.40 mg |
| gegebenenfalls HPMC Lack | 3.00 mg |

### Beispiel 8 (Vergleichsbeispiel)

Tablette enthaltend 50 mg N-[5-(Aminosulfonyl)-4-methyl-1,3-thiazol-2-yl]-N-methyl-2-[4-(2-pyridinyl)-phenyl]acetamid als mikronisierter Wirkstoff, Wirkstoffgehalt ca. 54% (bezogen auf unlackierte Tablette) und Benzoesäure in einem molaren Verhältnis von 100%:

| | |
|---|---|
| N-[5-(Aminosulfonyl)-4-methyl-1,3-thiazol-2-yl]-N-methyl-2-[4-(2-pyridinyl)-phenyl]acetamid, mikronisiert | 50.00 mg (0.124 mmol) |
| Polyvinylpyrrolidon 25 | 5.00 mg |
| Mikrokristalline Cellulose | 1.47 mg |
| Benzoesäure | 15.20 mg (0.124 mmol) |
| Croscarmellose Natrium | 20.00 mg |
| Magnesiumstearat | 0.93 mg |
| Tween 80 | 0.40 mg |
| gegebenenfalls HPMC Lack | 3.00 mg |

Zur Bestimmung der Lagerstabilität der Tabletten werden diese wie allgemein üblich in offenen Glasgefäßen bei den angegeben Temperaturen bzw. Luftfeuchten eingelagert. Nach bestimmten Zeiten wird die chemische und physikalische Stabilität der Tabletten getestet. Dieses Verfahren ist in der industriellen Entwicklung von Tabletten zum Einsatz als Arzneimittel allgemein üblich und dem Fachmann bekannt.

### Beschreibung der Abbildungen

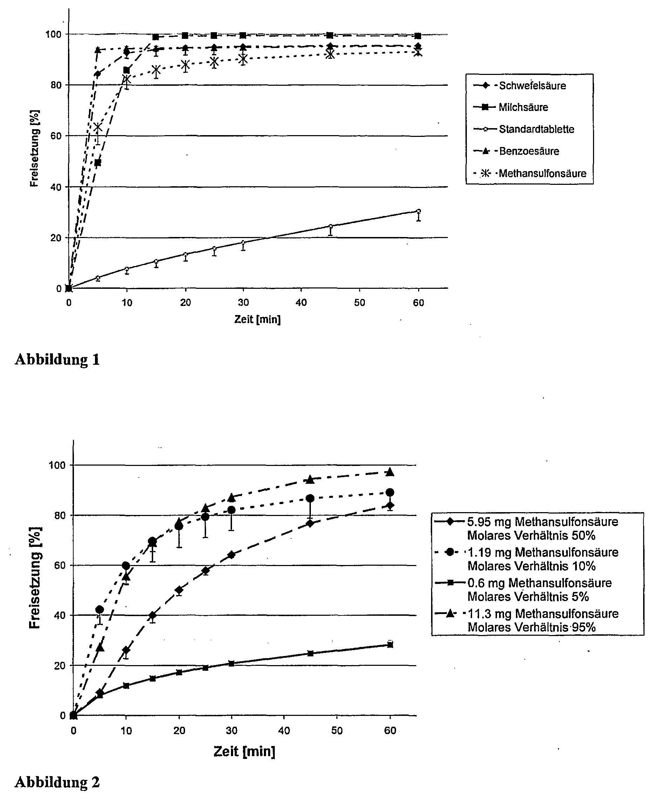

**Abbildung 1** zeigt den Vergleich der Freisetzung von N-[5-(Aminosulfonyl)-4-methyl-1,3-thiazol-2-yl]-N-methyl-2-[4-(2-pyridinyl)-phenyl]acetamid aus Standardtabletten und Tabletten mit Säurezusatz in 0.1N Salzsäure bei 37°C.

| **Zeit [min]** | **Schwefelsäure Freisetzung [%]** | **Milchsäure Freisetzung [%]** | **Benzoesäure Freisetzung [%]** | **Methansulfonsäure Freisetzung [%]** | **Standardtablette Freisetzung [%]** |
|---|---|---|---|---|---|
| **0** | 0 | 0 | 0 | 0 | 0 |
| **5** | 84 | 49 | 94 | 63 | 6 |
| **10** | 93 | 86 | 94 | 82 | 10 |
| **15** | 94 | 99 | 94 | 86 | 13 |
| **20** | 95 | 99 | 95 | 88 | 16 |
| **25** | 95 | 99 | 95 | 89 | 19 |
| **30** | 95 | 99 | 95 | 90 | 21 |
| **45** | 95 | 99 | 95 | 92 | 28 |
| **60** | 96 | 99 | 95 | 93 | 34 |

Abbildung 1 zeigt deutlich die verbesserte Freisetzung von N-[5-(Aminosulfonyl)-4-methyl-1,3-thiazol-2-yl]-N-methyl-2-[4-(2-pyridinyl)-phenyl]acetamid aus Tabletten mit Säurezusatz.

**Abbildung 2** zeigt den Vergleich der Freisetzung von N-[5-(Aminosulfonyl)-4-methyl-1,3-thiazol-2-yl]-N-methyl-2-[4-(2-pyridinyl)-phenyl]acetamid aus Tabletten mit verschiedenen molaren Mengen Methansulfonsäure in 0.1N Salzsäure bei 37°C.

| **Zeit [min]** | **5% Methansulfonsäure Freisetzung [%]** | **10% Methan sulfonsäure Freisetzung [%]** | **50% Methansulfonsäure Freisetzung [%]** | **95% Methansulfonsäure Freisetzung [%]** |
|---|---|---|---|---|
| **0** | 0 | 0 | 0 | 0 |
| **5** | 8 | 42 | 9 | 27 |
| **10** | 12 | 60 | 26 | 56 |
| **15** | 15 | 70 | 40 | 69 |
| **20** | 17 | 76 | 50 | 77 |
| **25** | 19 | 79 | 58 | 83 |
| **30** | 21 | 82 | 64 | 87 |
| **45** | 25 | 87 | 77 | 94 |
| **60** | 28 | 89 | 84 | 97 |

Abbildung 2 zeigt deutlich die verbesserte Freisetzung von N-[5-(Aminosulfonyl)-4-methyl-1,3-thiazol-2-yl]-N-methyl-2-[4-(2-pyridinyl)-phenyl]acetamid aus Tabletten mit einem hohen molaren Anteil an Methansulfonsäure.

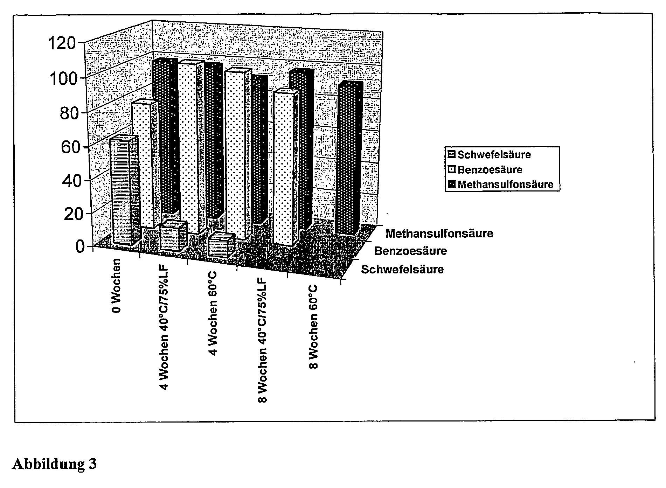

**Abbildung 3** zeigt den Vergleich der Freisetzung von N-[5-(Aminosulfonyl)-4-methyl-1,3-thiazol-2-yl]-N-methyl-2-[4-(2-pyridinyl)-phenyl]acetamid aus Tabletten nach 15 min [%] in Abhängigkeit von Lagerzeit und Lagerbedingungen.

| **Säure** | **0 Wochen** | **4 Wochen 40°C/75% Luftfeuchtigkeit** | **4 Wochen 60°C** | **8 Wochen 40°C/75% Luftfeuchtigkeit** | **8 Wochen 60°C** |
|---|---|---|---|---|---|
| **Schwefelsäure** | 63 | 14 | 10 | -- | -- |
| **Benzoesäure** | 78 | 104 | 101 | 91 | -- |
| **Methansulfonsäure** | 98 | 97 | 91 | 97 | 91 |

Abbildung 3 zeigt deutlich die verbesserte Lagerstabilität von N-[5-(Aminosulfonyl)-4-methyl-1,3-thiazol-2-yl]-N-methyl-2-[4-(2-pyridinyl)-phenyl]acetamid in Tabletten bei der Verwendung von Methansulfonsäure zur Herstellung der pharmazeutischen Formulierung.

## Patentansprüche

1. Pharmazeutische Zubereitung zur oralen Anwendung, die
N-[5-(Aminosulfonyl)-4-methyl-1,3-thiazol-2-yl]-N-methyl-2-[4-(2-pyridinyl)-phenyl]-acetamid oder ein Hydrat und/oder Solvat davon,
gegebenenfalls ein oder mehrere Trockenbindemittel,
mindestens ein Zerfallhilfsmittel,
mindestens ein Schmiermittel, und
gegebenenfalls ein Tensid enthält,
**dadurch gekennzeichnet, dass** die Zubereitung Methansulfonsäure enthält, die in einem stöchiometrischen Verhältnis von N-[5-(Aminosulfonyl)-4-methyl-1,3-thiazol-2-yl]-N-methyl-2-[4-(2-pyridinyl)phenyl]acetamid oder des Hydrates und/oder Solvates zur Säure von 1 zu 0.10 bis 1.10 eingesetzt wird, wobei das Acetamid und die Säure ein Salz bilden.

2. Zubereitung nach Anspruch 1, **dadurch gekennzeichnet, dass** die Zubereitung von 30 bis 1200 mg N-[5-(Aminosulfonyl)-4-methyl-1,3-thiazol-2-yl]-N-methyl-2-[4-(2-pyridinyl)-phenyl]acetamid oder ein Hydrat und/oder Solvat bezogen auf eine einzelne Tablette enthält.

3. Zubereitung nach Anspruch 1 oder 2, **dadurch gekennzeichnet, dass** das Trockenbindemittel mikrokristalline Cellulose ist.

4. Zubereitung nach einem der Ansprüche 1 bis 3, **dadurch gekennzeichnet, dass** das Zerfallhilfsmittel Croscarmellose-Natrium oder ein Gemisch aus Croscarmellose-Natrium und quervernetztes Polyvinylpyrrolidon (4: 1) ist.

5. Zubereitung nach einem der Ansprüche 1 bis 4, **dadurch gekennzeichnet, dass** das Schmiermittel Magnesiumstearat ist.

6. Tablettenzubereitung zur oralen Anwendung, die einen Kern aus der pharmazeutischen Zubereitung nach einem der Ansprüche 1 bis 5 und einen Lacküberzug umfasst.

7. Zubereitung nach einem der Ansprüche 1 bis 6, **dadurch gekennzeichnet, dass** sie N-[5-(Aminosulfonyl)-4-methyl-1,3-thiazol-2-yl]-N-methyl-2-[4-(2-pyridinyl)-phenyl]acetamid enthält.

8. Zubereitung nach einem der Ansprüche 1 bis 7, zur Anwendung in der Behandlung und/oder Prophylaxe von Erkrankungen, die durch Herpes-Viren, insbesondere Erkrankungen, die durch Herpes Simplex Viren hervorgerufen werden.

9. Verfahren zur Herstellung der pharmazeutischen Zubereitung zur oralen Anwendung nach einem der Ansprüche 1 bis 8, **dadurch gekennzeichnet, dass** N-[5-(Aminosulfonyl)-4-methyl-1,3-thiazol-2-yl]-N-methyl-2-[4-(2-pyridinyl)-phenyl]acetamid, dessen Hydrat und/oder Solvat, gegebenenfalls ein oder mehrere Trockenbindemittel und eine Säure wässrig granuliert werden, das Granulat anschließend mit mindestens einem Zerfallhilfsmittel und mindestens einem Schmiermittel und gegebenenfalls einem Netzmittel vermischt und gegebenenfalls tablettiert und lackiert wird.

## Claims

1. A pharmaceutical preparation for oral application, which contains
N-[5-(aminosulfonyl)-4-methyl-1,3-thiazol-2-yl]-N-methyl-2-[4-(2-pyridinyl)-phenyl]-acetamide or a hydrate and/or solvate thereof,
if applicable one or more dry binding agents,
at least one disintegration agent,
at least one lubricant, and
if applicable one surfactant,
**characterized in that** the preparation contains methanesulphonic acid, which is used in a stoichiometric ratio of N-[5-aminosulfonyl-4-methyl-1,3-thiazol-2-yl]-N-methyl-2-[4-(2-pyridinyl)phenyl]acetamide or of the hydrate and/or solvate to acid of 1 to 0.10 up to 1.10, wherein the acetamide and the acid form a salt.

2. The preparation according to claim 1, **characterized in that** the preparation of 30 to 1200 mg contains N-[5-(aminosulfonyl)-4-methyl-1,3-thiazol-2-yl]-N-methyl-2-[4-(2-pyridinyl)-phenyl]acetamide or a hydrate and/or solvate with reference to an individual tablet.

3. The preparation according to claim 1 or 2, **characterized in that** the dry binding agent is microcrystalline cellulose.

4. The preparation according to any one of the claims 1 to 3, **characterized in that** the disintegration agent is croscarmellose sodium or a mixture of croscarmellose sodium and cross-linked polyvinyl pyrrolidone (4:1).

5. The preparation according to any one of the claims 1 to 4, **characterized in that** the lubricant is magnesium stearate.

6. A preparation of tablets for oral application, which comprises a core from the pharmaceutical preparation according to any one of the claims 1 to 5 and a lacquer coating.

7. The preparation according to any one of the claims 1 to 6, **characterized in that** it contains N-[5-(aminosulfonyl)-4-methyl-1,3-thiazol-2-yl]-N-methyl-2-[4-(2-pyridinyl)-phenyl] acetamide.

8. The preparation according to any one of the claims 1 to 7, for application in the treatment and/or prophylaxis of diseases, which are caused by herpes viruses, in particular expectations, which result from herpes simplex viruses.

9. A method for manufacturing the pharmaceutical preparation for oral application according to any one of the claims 1 to 8, **characterized in that** N-[5-(aminosulfonyl)-4-methyl-1,3-thiazol-2-yl]-N-methyl-2-[4-(2-pyridinyl)-phenyl]acetamide, its hydrate and/or solvate, if applicable one or more dry binding agents and an acid are granulated aqueously, the granulate is subsequently mixed with at least a disintegration agent and at least one lubricant and, if applicable, a wetting agent, and if applicable tabletted and lacquered.

## Revendications

1. Préparation pharmaceutique pour application orale, contenant
N-[5-(aminosulfonyle)-4-méthyle-1,3-thiazol-2-yl]-N-méthyle-2-[4-(2-pyridinyle)-phényle]-acétamide ou un hydrate et/ou solvate de ce dernier,
si nécessaire un ou plusieurs liants à sec,
au moins un agent désintégrant,
au moins un lubrifiant, et
si nécessaire un agent tensioactif,
**caractérisée en ce que** la préparation contient de l'acide méthanesulfonique utilisée dans un rapport stoechiométrique de N-[5-(aminosulfonyle)-4-méthyle-1,3-thiazol-2-yl]-N-méthyle-2-[4-(2-pyridinyle)phényle]acétamide ou de l'hydrate et/ou du solvate à l'acide égal à 1 à 0,10 à 1,10, étant donné que l'acétamide et l'acide forment un sel.

2. Préparation selon la revendication 1, **caractérisée en ce que** la préparation de 30 à 1200 mg contient la N-[5-(aminosulfonyle)-4-méthyle-1,3-thiazol-2-yl]-N-méthyle-2-[4-(2-pyridinyle)-phényle]acétamide ou un hydrate et/ou solvate par rapport à un comprimé individuel.

3. Préparation selon la revendication 1 ou 2, **caractérisée en ce que** le liant à sec est la cellulose micro-cristalline.

4. Préparation selon l'une quelconque des revendications 1 à 3, **caractérisée en ce que** l'agent désintégrant est le sodium croscarmellose ou un mélange de sodium croscarmellose et pyrrolidone de polyvinyle à réticulation transversale (4:1).

5. Préparation selon l'une quelconque des revendications 1 à 4, **caractérisée en ce que** le lubrifiant est le stéarate de magnésium.

6. Préparation de comprimé pour application orale, comprenant un noyau de la préparation pharmaceutique selon l'une quelconque des revendications 1 à 5 et un revêtement vernis.

7. Préparation selon l'une quelconque des revendications 1 à 6, **caractérisée en ce qu'**elle contient la N-[5-(aminosulfonyle)-4-méthyle-1,3-thiazol-2-yl]-N-méthyle-2-[4-(2-pyridinyle)-phényle]-acétamide.

8. Préparation selon l'une quelconque des revendications 1 à 7, pour application dans le traitement et/ou la prophylaxie de maladies causées par des virus de l'herpès, en particulier les attentes provoquées par les virus de l'herpès simplex.

9. Procédé de production de la préparation pharmaceutique pour application orale selon l'une quelconque des revendications 1 à 8, **caractérisé en ce que** la N-[5-(aminosulfonyle)-4-méthyle-1,3-thiazol-2-yl]-N-méthyle-2-[4-(2-pyridinyle)-phényle]acétamide, son hydrate et/ou solvate, si nécessaire un ou plusieurs liants à sec et un acide sont granulés par voie aqueuse, le granulé est ensuite mélangé avec au moins un agent désintégrant et au moins un lubrifiant et, si nécessaire, un agent mouillant et, si nécessaire, préformé en comprimé et laqué.
